# EUROPEAN PATENT SPECIFICATION

(11) **EP 2 630 929 B1**
(45) Date of publication and mention of the grant of the patent: **22.06.2016**
(21) Application number: 13156300.9
(22) Date of filing: 22.02.2013
(51) Int. Cl.: A61B 17/34

(54) **Multi-portion wound protector**
Wundschutz mit mehreren Teilen
Dispositif protecteur de blessure multi-portions

(30) Priority: 23.02.2012 US 201261602099 P; 31.01.2013 US 201313755212
(43) Date of publication of application: 28.08.2013
(73) Proprietor: Covidien LP, Mansfield, MA 02048 (US)
(72) Inventor: Smith, Robert C., Middlefield, CT 06455 (US)
(74) Representative: Soames, Candida Jane

(56) References cited:
- WO-A1-01/91652
- WO-A1-2004/030547
- US-A- 6 033 428
- US-A1- 2003 139 767

## Description

### BACKGROUND

### Technical Field

The present disclosure relates generally to surgical apparatuses for use in minimally invasive surgical procedures, such as endoscopic and/or laparoscopic procedures, and more particularly, relates to a surgical apparatus that allows human hands or multiple surgical instruments to be inserted through a single opening through body tissue.

### Description of Related Art

Today, many surgical procedures are performed through small incisions in the skin, as compared to large incisions that are typically required in traditional procedures, in an effort to reduce trauma to the patient and reduce the patient's recovery time. Generally, such procedures are referred to as "endoscopic", unless performed on the patient's abdomen, in which case the procedure is referred to as "laparoscopic." Throughout the present disclosure, the term "minimally invasive" should be understood to encompass both endoscopic and laparoscopic procedures.

During a typical minimally invasive procedure, surgical instruments, such as endoscopes, graspers, staplers and forceps, are inserted into the patient's body through the incision in tissue. In general, prior to the introduction of the surgical object into the patient's body, insufflation gas is supplied to the target surgical site to enlarge its surrounding area and create a larger, more accessible work area. This is accomplished with a substantially fluid-tight seal that maintains the insufflation gas at a pressure sufficient to inflate the target surgical site.

In certain minimally invasive procedures, when a certain size of a specimen needs to be removed from the patient's body, a relatively large incision needs to be made. Some surgeons use the relatively large incision to have their hands in the operative field to aid the removal or other surgical procedures. This hand-assist technique reduces operative time significantly versus the typical minimally invasive approach, and also gives the surgeons more options in dealing with unexpected adverse events, such as uncontrolled bleeding. While a surgeon may place his/her hand in the operative field at some time during the procedure, the surgeon still needs to work with surgical instruments at other time during the same procedure, insufflation gas therefore must be continuously maintained at the target surgical site throughout the entire procedure.

Document WO 01/91652, which forms the basis of the preamble of claim 1, discloses an invaginator apparatus for removal of a body specimen from the body cavity comprising an outer sleeve to be placed in an incision and an invertable inner sleeve to collect the specimen.

It is desirable to have an access device to accommodate human hands and surgical instruments of different dimensions and form a substantial sealing relationship thereto to inhibit the escape of insufflation gas. It is also desirable for the access device to have an opening with an expandable nature to easily conform with the dimensions of human hands and surgical instruments inserted therein.

The existing access devices in the prior art such as wound retractors are generally known for permitting hand-assist procedures, but are also known for their drawbacks such as failure to inhibit escape of insufflation gas when instruments of dimensions smaller than the hands are operated therethrough.

Based on the above, a continuing need exists for an access device with increased versatility and enhanced sealing features to accommodate human hands and surgical instruments.

### SUMMARY

Disclosed herein is a surgical apparatus for positioning within a tissue tract accessing an underlying body cavity. The surgical apparatus includes a flexible member having an open proximal end, a closed distal end and a passage extending therebetween. The surgical apparatus further includes a string extending through the passage of the flexible member with a first end extending proximally beyond the open proximal end and a second end attached to the closed distal end and.

In one embodiment, the surgical apparatus includes an anchor member attached to the open proximal end of the flexible member. The anchor member is made from a material more rigid than that of the flexible material. For instance, the anchor member is made from a rigid or semi rigid material. The anchor member defines a passage for reception of objects therethrough. The passage of the anchor member is also configured to receive an access device therein.

In some embodiments, the closed distal end of the flexible member exhibits a generally conical shape.

In certain embodiments, an application of force on the string in a proximal direction causes the flexible member to invert and propagate in the proximal direction, and expose the closed distal end of the flexible member proximally beyond the anchor member.

Also described is an exemplary method of accessing an underlying body cavity through a tissue tract. The method includes positioning a surgical apparatus within the tissue tract. The surgical apparatus includes a flexible member defining an open proximal end, a closed distal end, and a passage extending therebetween. The surgical apparatus also includes a string extending through the passage of the flexible member with a first end extending proximally beyond the open proximal end and a second end attached to the closed distal end.

The exemplary method also includes pulling the string in a proximal direction to invert the flexible member. Additionally, the method includes removing a portion of the closed distal end of the flexible member resulting in an opening to permit objects therethrough.

Further, the surgical apparatus includes an anchor member attached to the open proximal end of the flexible member. The exemplary method includes inserting an access device into a passage defined in the anchor member.

### DESCRIPTION OF THE DRAWINGS

The above and other aspects, features, and advantages of the present disclosure will become more apparent in light of the following detailed description when taken in conjunction with the accompanying drawings in which:
**FIG. 1** is a front perspective view of a surgical apparatus in accordance with the principles of the present disclosure illustrating a surgical apparatus positioned relative to the tissue;
**FIG. 2** is an exploded view of the surgical apparatus of FIG. 1 illustrating an anchor member and a flexible member;
**FIG. 3** is a side cross-sectional view of the surgical apparatus of FIG. 1 illustrating the surgical apparatus positioned above the tissue;
**FIG. 4** is a side cross-sectional view of the surgical apparatus of FIG. 3 illustrating the surgical apparatus disposed within the tissue;
**FIG. 5** is a side cross-sectional view of the surgical apparatus of FIG. 4 illustrating removing a portion of the flexible member of the surgical apparatus while the flexible member is inverted;
**FIG. 6** is a side cross-sectional view of the surgical apparatus of FIG. 5 illustrating the remaining portion of the flexible member disposed within the tissue;
**FIG. 7** is a side cross-sectional view of the surgical apparatus of FIG. 6 illustrating an access device positioned above the surgical apparatus; and
**FIG. 8** is a side cross-sectional view of the surgical apparatus of FIG. 7 illustrating the access device disposed within the surgical apparatus.

### DETAILED DESCRIPTION

Particular embodiments of the present disclosure will be described herein with reference to the accompanying drawings. As shown in the drawings and as described throughout the following description, and as is traditional when referring to relative positioning on an object, the term "proximal" or "trailing" refers to the end of the apparatus that is closer to the user and the term "distal" or "leading" refers to the end of the apparatus that is farther from the user. In the following description, well-known functions or constructions are not described in detail to avoid obscuring the present disclosure in unnecessary detail.

One type of minimal invasive surgery described herein employs a device that facilitates multiple instrument access through a single incision. This is a minimally invasive surgical procedure, which permits a user to operate through a single entry point, typically the patient's navel. Additionally, the presently disclosed device may be used in a procedure where a naturally occurring orifice (e.g. vagina or anus) is the point of entry to the surgical site. The disclosed procedure involves insufflating the body cavity and positioning a portal member within, e.g., the navel of the patient. Instruments including an endoscope and additional instruments such as graspers, staplers, forceps or the like may be introduced within a portal member to carry out the surgical procedure. An example of such a surgical portal is disclosed in U.S. patent application Serial No. 12/244,024, Pub. No. US 2009/0093752 A1, filed October 2, 2008.

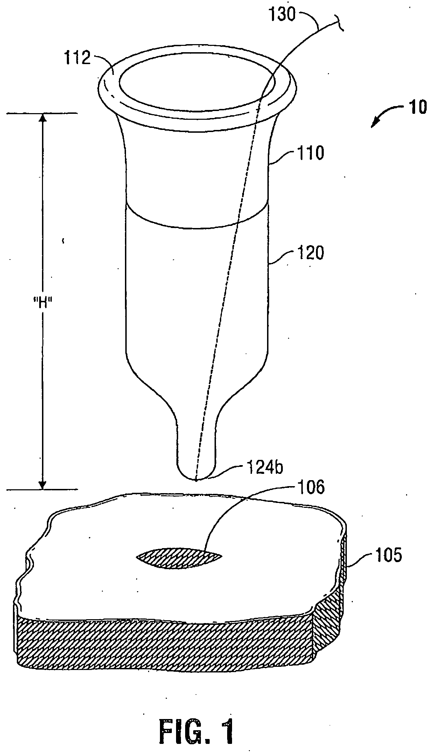

Referring now to the drawings, in which like reference numerals identify identical or substantially similar parts throughout the several views, **FIG.** 1 illustrates a surgical apparatus **10** in accordance with the principles of the present disclosure. The surgical apparatus **10** is adapted for insertion in a tissue opening 106 within a tissue tract **105,** e.g., through the abdominal or peritoneal lining in connection with a laparoscopic surgical procedure. The surgical apparatus **10** will be described in greater detail hereinbelow.

As shown in **FIG.** 1, the surgical apparatus **10** may define a generally cylindrical shape with a closed distal end. However, it is contemplated that the surgical apparatus **10** may define other configurations both prior and subsequent to insertion within the tissue tract **105.**

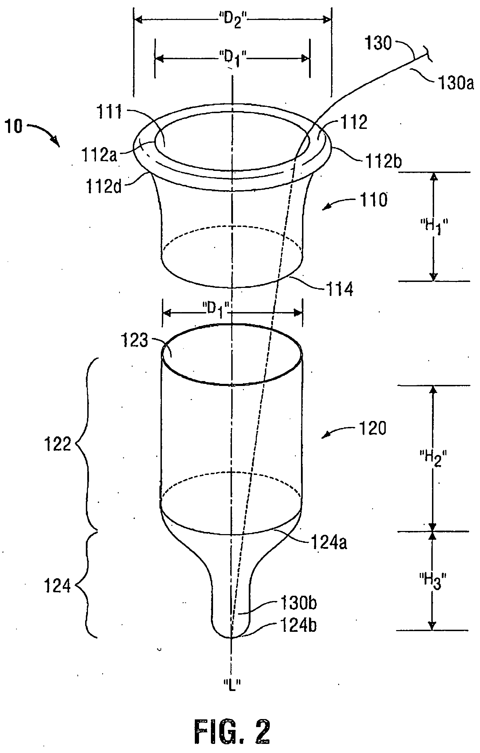

With reference to **FIG. 2****,** the surgical apparatus 10 defines a longitudinal axis "L", and includes an anchor member (or a proximal member) 110 and a flexible member (or a distal member) 120 which are axially aligned along the longitudinal axis "L," with the anchor member 110 mounted on top of or mounted proximally with respect to the flexible member 120. The anchor member 110 exhibits a generally cylindrical configuration and includes a proximal end 112 and a distal end 114. It is envisioned that the anchor member 110 may exhibit other configurations. The anchor member 110 defines a longitudinal passage 111 extending from the proximal end 112 to the distal end 114 and having a diameter "D1" in its radial dimension. In one embodiment, the anchor member 110 has a uniform radial dimension (e.g. "D1") along its length. It is also contemplated that the anchor member 110 may exhibit a tapering configuration with a gradually changing radial dimension between the proximal end 112 and the distal end 114.

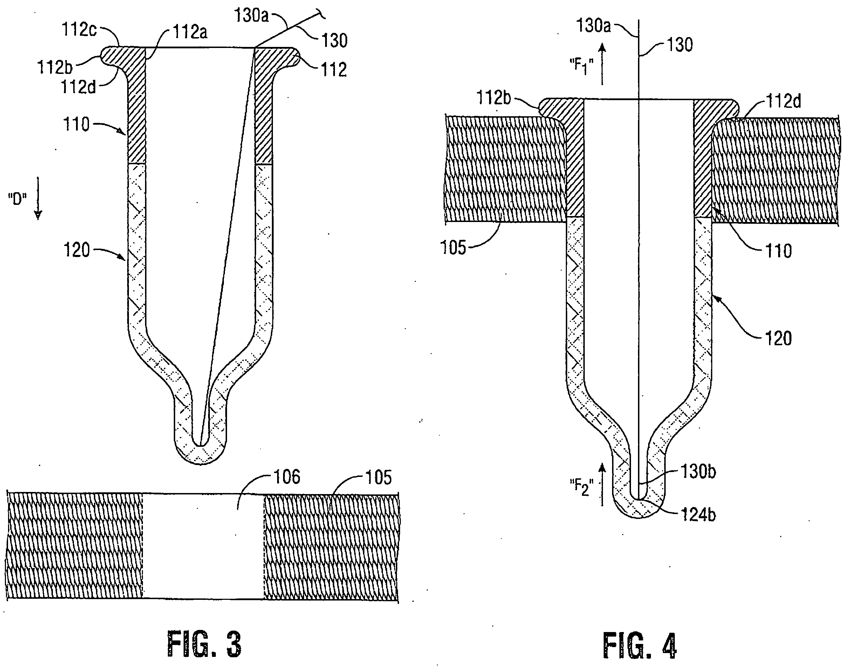

The proximal end 112 of the anchor member 110 is in the shape of an annular flange 112. As seen in FIG. 3, the annular flange 112 includes an inner wall 112a, an outer wall 112b, a proximal surface 112c and a distal surface 112d. As illustrated in FIG. 2, the inner wall 112a of the annular flange 112 defines an inner diameter identical to the diameter "D1" of the longitudinal passage 111. The annular flange 112 is configured to be disposed exteriorly outside of the tissue opening 106. Specifically, as illustrated in FIG. 2, the outer wall 112b is configured to have an outer diameter "D2," which is larger than "D1" and also significantly greater than the size of the tissue opening 106. As illustrated in FIG. 4, when the anchor member 110 is disposed in the tissue opening 106, the distal surface 112d of the annular flange 112 abuts the upper side of the tissue tract 105, and the outer wall 112b of the annular flange 112 inhibits the annular flange 112 from entering the tissue opening 106 and facilitates retraction of the tissue opening 106. It is envisioned that when the anchor member 110 is disposed in the tissue opening 106, the anchor member 110 forms a substantial sealing relation with the tissue tract 105.

As illustrated in FIG. 2, the anchor member 110 defines an insertion length "H1" which is a distance measured from the distal surface 112d of the annular flange 112 to the distal end 114 of the anchor member 110. The insertion length "H1" represents the length of the anchor member 110 that is insertable into the tissue opening 106. The insertion length "H1" is also the minimum length required to anchor the surgical apparatus 10 within any type of tissue tract 105.

With continued reference to FIG. 2, the flexible member 120 of the surgical apparatus 10 includes a uniform portion (or a proximal portion) 122 and a narrow portion (or a distal portion) 124. The uniform portion 122 exhibits a generally cylindrical shape with a longitudinal passage 123 defined therein. The longitudinal passage 123 defines a uniform radial dimension along its length, and its radial dimension is identical to that of the longitudinal passage 111. The uniform portion 122 defines an insertion length "H2" which is insertable into the tissue opening 106. The insertion length "H2" is identical to the entire longitudinal length of the uniform portion 122. The insertion length "H2" of the uniform portion 122 equals to or is greater than the insertion length "H1" of the anchor member 110.

The narrow portion 124 of the flexible member 120 defines a diameter that varies along the longitudinal axis "L." In one embodiment, the narrow portion 124 defines a diameter gradually decreasing in a distal direction along the longitudinal axis "L."

In a certain embodiment, the narrow portion 124 exhibits a generally conical configuration, as seen in FIG. 2. The narrow portion 124 has a circular base 124a immediately connected to the uniform portion 122. The circular base 124a defines a diameter "D1" which is identical to that of the longitudinal passage 123. Further, the narrow portion 124 has an apex end (or a distal-most end) 124b, which is closed and has an almost negligible diameter. The diameter of the narrow portion 124 gradually decreases along the longitudinal axis "L" from the circular base 124a, where the diameter is maximized, to the apex end 124b, where the diameter is minimized. The narrow portion 124 defines an insertion length "H3" insertable into the tissue opening 106. The insertion length "H3" is a distance measured from the circular base 124a to the apex end 124b.

As seen in FIG. 1, the surgical apparatus 10 defines an insertion length "H" which is identical to the combined insertion lengths of the anchor member 110 (i.e., "H1"), the uniform portion 122 (i.e., "H2"), and the narrow portion 124 (i.e., "H3").

In a certain embodiment, the anchor member 110 is made of a rigid or semi rigid material such as plastic or rubber, which is able to establish a sealing relation with the tissue tract 105. The flexible member 120 may be made from a semi-resilient, disposable, compressible and flexible type (e.g. rubber or sponge) material, for example, but not limited to, a suitable foam, gel material, or soft rubber having sufficient compliance to form a seal about one or more surgical objects, and also establish a sealing relation with the tissue tract 105 and with the surgical object. In one embodiment, the foam includes a polyisoprene material. The resilient nature of the flexible member 120 provides an easy insertion and removal of the surgical apparatus 10 through the tissue 105.

In some embodiments, the anchor member 110 is made of a material more rigid than that of the flexible member 120.

In one embodiment, the anchor member 110 is an integrated part of the flexible member 120. For instance, the anchor member 110 is permanently attached to the flexible member 120 by glue, welding or by an overmolding process.

In another embodiment, the anchor member 110 is detachably connected to the flexible member 120.

The surgical apparatus 10 further includes a string 130. The string 130 is of a length substantially greater than the insertion length "H" of the surgical apparatus 10. In a certain embodiment, the string 130 is an integrated member of the flexible member 120. For instance, the string 130 is permanently attached to the flexible member 120 by glue, welding or by an overmolding process.

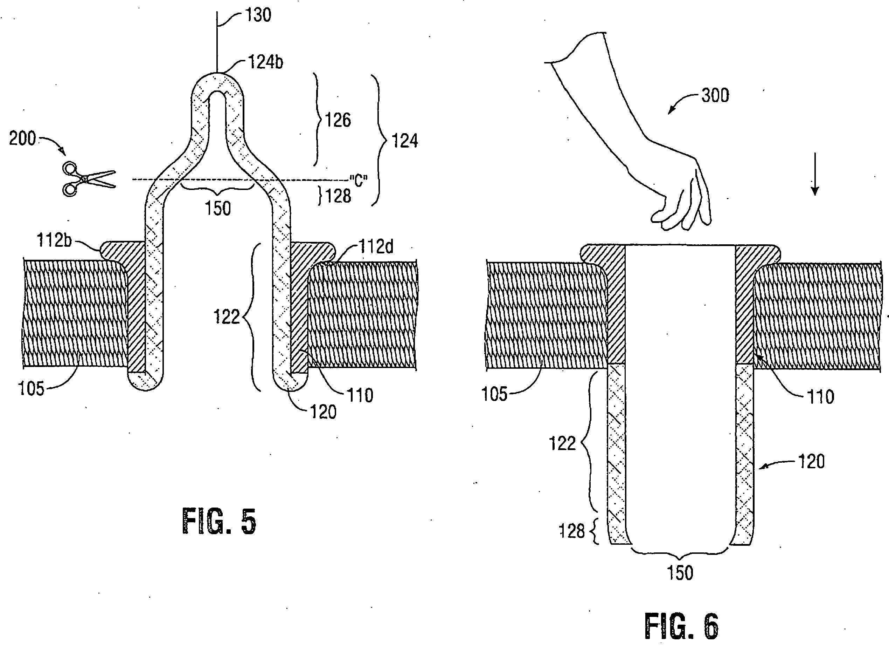

As seen in FIG. 2, the string 130 has a first end 130a freely disposed outside of the surgical apparatus 10, and a second end 130b connected to an inner wall of the narrow portion 124. In one embodiment, the second end 130b is disposed interiorly of the narrow portion 124 and attached to the apex end 124b. As seen in FIG. 4, a first force "F1" acting on the first end 130a of the string 130 induces a second force "F2" on the apex end 124b of the surgical apparatus 10. As seen in FIG. 5, the force "F2" causes the apex end 124b to invert and propagate in the proximal direction through the longitudinal passage 123 of the flexible member 120, and subsequently through the longitudinal passage 111 of the anchor member 110. Due to the flexible nature of the flexible member 120, the flexible member 120 is inverted under the application of force "F2." Once the flexible member 120 is completely inverted as illustrated in FIG. 5, the force "F2" or the force "F1" which induced the force "F2" is removed. In its completely inverted state, the flexible member 120 is folded inwardly into the anchor member 110 to the extent that the narrow portion 124 is completely exposed above the tissue tract 105. As seen in FIG. 5, the interior of the anchor member 110 is completely overlaid by at least a portion of the uniform portion 122. When the flexible member 120 is completely inverted, the anchor member 110 remains in a sealing relationship with the tissue tract 105, and the distal surface 112d of the annular flange 112 remains in an abutting relationship with the upper side of the tissue tract 105.

With continued references to FIGS. 5-6, once the narrow portion 124 is exposed above the tissue tract 105, a part 126 of the narrow portion 124 may be removed to create an opening 150 in the remaining part 128 of the narrow portion 124. For instance, a surgeon may use a scissor to cut off a part 126 of the narrow portion 124 along the axis "C" as illustrated in FIG. 5. The opening 150 is of a size that allows a human hand 300 to pass therethrough. The size of the opening 150 varies depending on the size of the part 126 removed from the narrow portion 124. For instance, the size of the opening 150 may vary from an almost negligible diameter, if only the apex end 124b of the narrow portion 124 is removed, to a maximum diameter "D1," when the narrow portion 124 in its entirety is removed. The surgeon selectively determines the size of the opening 150 to be created based on the size of the surgeon's hand 300 that needs to pass through the opening 150. It is envisioned that the opening 150 forms a sealing relationship with the hand 300 passed therethrough to facilitate hand-assisted procedures. It is also envisioned that the opening 150 has an expandable nature to sealingly accommodate hands or instruments with radial dimensions greater than the size of the opening 150.

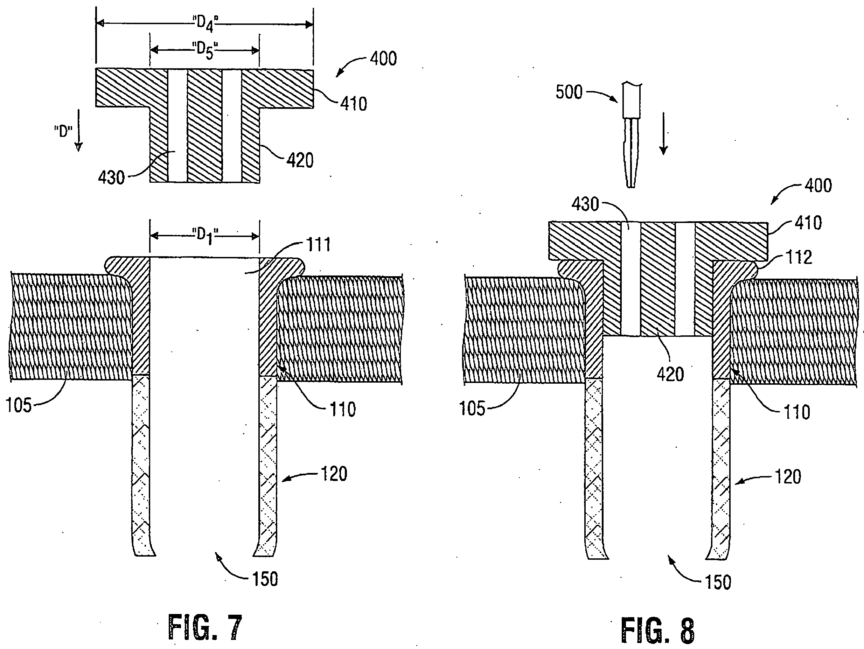

The surgical apparatus 10 may also be used in conjunction with an intermediate access device (e.g. a portal member 400 shown in FIGS. 7 and 8) to receive surgical objects having radial dimensions smaller than that of a hand. The longitudinal passage 111 of the surgical apparatus 10 is adapted to receive the portal member 400 such as that disclosed in U.S. patent application Serial No. 12/244,024, Pub. No. US 2009/0093752 A1, filed October 2, 2008. The portal member 400 defines at least one longitudinal passage 430 between its proximal end 410 and its distal end 420 for reception of a surgical object 500 therethrough in a substantially sealing relationship. It is envisioned that the surgical object 500 has a radial dimension substantially smaller than that of a human hand. With reference to FIG. 7, the portal member 20 defines a relatively large radial dimension "D4" at its proximal end 410 and defines a relatively small radial dimension "D5" at its distal end 420. The radial dimension "D5" of the distal end 420 equals to or is slightly greater than the radial dimension "D1" of the longitudinal passage 111 such that the distal end 420 can fit snugly within the longitudinal passage 111 and forms a sealing engagement with the longitudinal passage 111. The radial dimension "D4" of the proximal end 410 is significantly larger than the radial dimension "D1" of the longitudinal passage 111. Accordingly, the longitudinal passage 111 prevents the entry of the proximal end 410.

In operation, the surgeon inserts the surgical apparatus 10, as illustrated in FIG. 3, into the tissue opening 106 of the tissue tract 105 with the first end 130a of the string 130 extending proximally beyond the annular flange 112 of the surgical apparatus. The surgical apparatus 10 advances distally into the tissue opening 106 until the distal surface 112d of the annular flange 112 abuts the upper side of the tissue tract 105. Second, the surgeon pulls the string 130 proximally as indicated in FIGS. 4-5 to expose the narrow portion 124 proximally beyond the annular flange 112 of the surgical apparatus 10. Third, with reference to FIGS. 5-6, the surgeon removes a part 126 of the narrow portion 124 or the entire narrow portion 124 to create an opening 150 which enables the surgeon to perform hand-assist procedures therethrough. Fourth, when typical minimally invasive procedures involving surgical instruments 500 of small radial dimensions are desired, the surgeon may mount the portal member 400 into the longitudinal passage 111 of the surgical apparatus 10 for receiving the surgical instruments 500.

In use, the same surgical apparatus 10 facilitates both hand-assisted procedures as well as typical minimally invasive procedures. The surgical apparatus 10 can sealingly engage human hands and large instruments of various dimensions, as well as sealingly engage small surgical instruments via an intermediate access device (e.g. portal member 400). In the first scenario, the surgeon creates an opening 150 in the surgical apparatus 10 at real time to accommodate the surgeon's hand or an instrument of similar dimension. A large opening can be created to sealingly engage a large hand or an instrument of a similar dimension; and on the other hand, a small opening can be created to sealingly engage a small hand or an instrument of a similar dimension. In the second scenario, when an instrument of a dimension relatively smaller than the surgeon's hand is desired during the procedure, the surgical apparatus 10 readily accommodates an intermediate access device (e.g. portal member 400) for receiving the relatively small instrument. The intermediate access device 400 is configured to form a sealing relation with the surgical apparatus 10. The intermediate access device 400 is also configured to sealingly receive instruments of dimensions relatively smaller than that of human hands. In both scenarios, the surgical apparatus 10 forms a substantial sealing relationship with the tissue tract 105, thereby preventing the escape of insufflation gas.

Further, the narrow portion 124 with its relatively small dimension provides easy insertion and removal of the surgical apparatus 10 through the tissue opening 106, thus, reducing the time required to place and/or displace the surgical apparatus 10 through the incisions during surgical operations and reducing tissue trauma.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Different embodiments of the disclosure may be combined with one another based on the particular needs of the patients to achieve optimal results of the surgical procedures. Those skilled in the art will envision other modifications within the scope of the claims appended hereto.

## Claims

1. A surgical apparatus for positioning within a tissue tract accessing an underlying body cavity, which comprises:
a flexible member (120) defining an open proximal end (123), a closed distal end (124b), and a passage extending there between;
a string (130) extending through the passage of the flexible member with a first end (130a) extending proximally beyond the open proximal end and a second end (130b) attached to the closed distal end;
**characterised by** an anchor member (110) attached to the open proximal end (123) of the flexible member (120) wherein the anchor member has a proximal flange, defines a passage for reception of objects therethrough, and is made from a material more rigid than that of the flexible member.

2. The surgical apparatus according to claim 1, wherein the anchor member (110) is made from a rigid or semi rigid material.

3. The surgical apparatus according to any preceding claim, wherein an application of force on the string (130) in a proximal direction inverts the flexible member such that the closed distal end extends proximally beyond the open proximal end of the flexible member.

4. The surgical apparatus according to claim 1, wherein the flexible member (120) is configured to invert and propagate proximally upon pulling the string (130) and to expose the closed distal end (124b) of the flexible member proximally beyond the anchor member.

5. The surgical apparatus according to any preceding claim, wherein the closed distal end (124b) of the flexible member exhibits a generally conical shape.

6. The surgical apparatus according to any preceding claim, wherein at least a part of the closed distal end (124b) of the flexible member is configured to be removed resulting in an opening to permit objects therethrough.

7. The surgical apparatus according to claim 1, wherein the anchor member (110) defines a passage for reception of objects therethrough.

8. The surgical apparatus according to claim 7, wherein the passage of the anchor member (110) is configured to receive an access device (400) therein.

9. The surgical apparatus according to claim 8, wherein the access device (400) forms a substantially sealing relationship with the anchor member.

10. The surgical apparatus according to any preceding claim, wherein the string (130) defines a length greater than that of the surgical apparatus.

## Patentansprüche

1. Eine chirurgische Vorrichtung zum Positionieren innerhalb eines Gewebetraktes, zugreifend auf einen darunterliegenden Körperhohlraum, welche aufweist:
ein flexibles Element (120), das ein offenes proximales Ende (123), ein geschlossenes distales Ende (124b) und einen sich dazwischen erstreckenden Durchlass definiert;
eine Schnur (130), die sich durch den Durchlass von dem flexiblen Element hindurch erstreckt, mit einem ersten Ende (130a), das sich proximal oberhalb des offenen proximalen Endes erstreckt, und einem zweiten Ende (130b), das an dem geschlossenen distalen Ende befestigt ist;
**gekennzeichnet durch** ein Ankerelement (110), das an dem offenen proximalen Ende (123) von dem flexiblen Element (120) befestigt ist, wobei das Ankerelement einen proximalen Flansch hat, einen Durchlass zum dort hindurch Empfangen von Objekten definiert und aus einem Material hergestellt ist, das starrer als das von dem flexiblen Element ist.

2. Die chirurgische Vorrichtung gemäß Anspruch 1, wobei das Ankerelement (110) aus einem starren oder halb-starren Material hergestellt ist.

3. Die chirurgische Vorrichtung gemäß einem vorangegangenen Anspruch, wobei eine Anwendung von Kraft auf die Schnur (130) in einer proximalen Richtung das flexible Element invertiert, so dass sich das geschlossene distale Ende proximal oberhalb des offenen proximalen Endes von dem flexiblen Element erstreckt.

4. Die chirurgische Vorrichtung gemäß Anspruch 1, wobei das flexible Element (120) konfiguriert ist zum Invertieren und proximalen Ausbreiten auf ein Ziehen der Schnur (130) und zum Exponieren des geschlossenen distalen Endes (124b) von dem flexiblen Element proximal oberhalb des Ankerelements.

5. Die chirurgische Vorrichtung gemäß einem vorangegangenen Anspruch, wobei das geschlossene distale Ende (124b) von dem flexiblen Element eine weitgehend konische Gestalt aufweist.

6. Die chirurgische Vorrichtung gemäß einem vorangegangenen Anspruch, wobei wenigstens ein Teil von dem geschlossenen distalen Ende (124b) von dem flexiblen Element konfiguriert ist, um entfernt zu werden, was zu einer Öffnung führt, um Objekte dort hindurch zu erlauben.

7. Die chirurgische Vorrichtung gemäß Anspruch 1, wobei das Ankerelement (110) einen Durchlass zum Empfang von Objekten dort hindurch definiert.

8. Die chirurgische Vorrichtung gemäß Anspruch 7, wobei der Durchlass von dem Ankerelement (110) konfiguriert ist zum darin Empfangen einer Zugangsvorrichtung (400).

9. Die chirurgische Vorrichtung gemäß Anspruch 8, wobei die Zugangsvorrichtung (400) eine im Wesentlichen dichtende Beziehung mit dem Ankerelement bildet.

10. Die chirurgische Vorrichtung gemäß einem vorangegangenen Anspruch, wobei die Schnur (130) eine Länge definiert, die größer als die von der chirurgischen Vorrichtung ist.

## Revendications

1. Appareil chirurgical pour positionner dans une voie tissulaire accédant à une cavité corporelle sous-jacente, qui comprend :
un élément souple (120) définissant une extrémité proximale ouverte (123), une extrémité distale fermée (124b) et un passage s'étendant entres elles ;
une ficelle (130) s'étendant à travers le passage de l'élément souple avec une première extrémité (130 a) qui s'étend de manière proximale au-delà de l'extrémité proximale ouverte et une seconde extrémité (130b) fixée à l'extrémité distale fermée ;
**caractérisé par** un élément d'ancrage (110) fixé à l'extrémité proximale ouverte (123) de l'élément souple (120), dans lequel l'élément d'ancrage présente une bride proximale, définit un passage pour la réception d'objets à travers celle-ci et est constitué d'un matériau plus rigide que celui de l'élément souple.

2. Appareil chirurgical selon la revendication 1, dans lequel l'élément d'ancrage (110) est constitué d'un matériau rigide ou semi-rigide.

3. Appareil chirurgical selon l'une quelconque des revendications précédentes, dans lequel l'application d'une force à la ficelle (130) dans une direction proximale inverse l'élément souple de sorte que l'extrémité distale fermée s'étende de manière proximale au-delà de l'extrémité proximale ouverte de l'élément souple.

4. Appareil chirurgical selon la revendication 1, dans lequel l'élément souple (120) est configuré pour s'inverser et se déployer de manière proximale lors de la traction sur la ficelle (130) et pour exposer l'extrémité distale fermée (124b) de l'élément souple de manière proximale au-delà de l'élément d'ancrage.

5. Appareil chirurgical selon l'une quelconque des revendications précédentes, dans lequel l'extrémité distale fermée (124b) de l'élément souple présente une forme générale conique.

6. Appareil chirurgical selon l'une quelconque des revendications précédentes, dans lequel au moins une partie de l'extrémité distale fermée (124b) de l'élément souple est configurée pour être retirée, ce qui entraîne l'apparition d'une ouverture pour pouvoir y faire passer des objets.

7. Appareil chirurgical selon la revendication 1, dans lequel l'élément d'ancrage (110) définit un passage pour y recevoir des objets.

8. Appareil chirurgical selon la revendication 7, dans lequel le passage de l'élément d'ancrage (110) est configuré pour y recevoir un dispositif d'accès (400).

9. Appareil chirurgical selon la revendication 8, dans lequel le dispositif d'accès (400) forme une relation sensiblement étanche avec l'élément d'ancrage.

10. Appareil chirurgical selon l'une quelconque des revendications précédentes, dans lequel la ficelle (130) définit une longueur supérieure à celle de l'appareil chirurgical.
